# EUROPEAN PATENT SPECIFICATION

(11) **EP 0 760 665 B1**
(45) Date of publication and mention of the grant of the patent: **12.12.2001**
(21) Application number: 95920842.2
(22) Date of filing: 18.05.1995
(51) Int. Cl.: A61K 31/55

(54) **COMPOSITIONS CONTAINING 5HTIA AND 5HTID ANTAGONISTS**
ZUSAMMENSETZUNG VON 5HTIDA UND 5HTID ANTAGONISTEN
COMPOSITIONS CONTENANT DES ANTAGONISTS DU TYPE 5HTIA ET 5HTID

(30) Priority: 25.05.1994 GB 9410512
(43) Date of publication of application: 12.03.1997
(73) Proprietor: SMITHKLINE BEECHAM PLC, Brentford, Middlesex TW8 9EP (GB)
(72) Inventor: JONES, Brian, John SmithKline Beecham, Harlow Essex CM19 5AD (GB); ROUTLEDGE, Carol SmithKline Beecham, Harlow Essex CM19 5AD (GB)
(74) Representative: Waters, David Martin, Dr.
(86) International application number: EP9501916
(87) International publication number: WO9531988

(56) References cited:
- EP-A- 0 533 267
- WO-A-94/03444

## Description

The present invention relates to novel combinations of compounds, pharmaceutical compositions containing them, and their use in therapy.

EPA 0 533 266/7/8 disclose a series of benzanilide derivatives which are said to possess 5HT_{1D} receptor antagonist activity. These compounds are said to be of use in the treatment of various CNS disorders, including depression. WO94/03444 discloses a series of phenyl piperazine derivatives which are said to be 5HT_{1A} antagonists. These compounds are also said to be of use in the treatment of various CNS disorders, including depression. The human 5HT_{1D} receptor is known to be encoded by two distinct genes initially designated 5HT_{1Dα} and 5HT_{1Dβ} and subsequently redesignated as 5HT_{1D} and 5HT_{1B} respectively (P.R. Hartig et al, Trends in Pharmacological Science, 1996, **17**, 103 - 105). All instances of the term "5HT_{1D}" are to be interpreted herein as meaning "5HT_{1D} and/or 5HT_{1B}" in accordance with this redesignation.

It has now surprisingly been found that administration of a combination of a 5HT_{1D} antagonist and a 5HT_{1A} antagonist is likely to be much more effective in treating CNS disorders than administration of a single 5HT_{1D} or 5HT_{1A} antagonist.

In a first aspect the present invention therefore provides a pharmaceutical composition which comprises:
- a compound having 5HT_{1D} antagonist activity;
- a compound having 5HT_{1A} antagonist activity; and
- a pharmaceutically acceptable carrier.

It will be understood that compounds having 5HT_{1D} or 5HT_{1A} activity can usually be isolated in salt form and the invention extends to compositions in which the compounds are in salt form. Preferred salts are pharmaceutically acceptable salts, for example acid addition salts such as hydrochlorides, hydrobromides, phosphates, acetates, fumarates, maleates, tartrates, citrates, oxalates, methanesulphonates and p-toluenesulphonates.

The invention also extends to compositions in which the compounds are in stereoisomeric or tautomeric forms.

Preferred 5HT_{1D} antagonists include those disclosed in EPA 0 533 266/7/8, in particular N-[4-methoxy-3-(4-methyl- 1-piperazinyl)phenyl]-2'-methyl-4'(5-methyl-1,2,4-oxadiazol-3-yl)[1,1'-biphenyl]-4-carboxamide. Other preferred 5HT_{1D} antagonists include those compounds disclosed in WO 95/04729, WO 95/06044, WO 95/06644 and WO 95/06637.

Preferred 5HT_{1A} antagonists include those disclosed in WO 94/03444, in particular (+)-2,3,4,5,6,7-hexahydro-1-(4-(1-(1,2,3,6-tetrahydro-4-(2-methoxyphenyl)pyridyl))-2-phenyl-butyryl)-1H-azepine. Other preferred 5HT_{1A} antagonists are 1-(1H-indol-4-yloxy)-3-[(1-methylethyl)amino]-2-propanol, (*S*)-5-fluoro-8-hydroxy-2-(dipropylamino)-tetralin, N-tert-butyl 3-4-(2-methoxyphenyl) piperazin-1-yl-2-phenylpropanamide dihydrochloride and (N-(2-(4-(2-methoxyphenyl)-1-piperazinyl)ethyl)-N-(2-pyridynyl)cyclohexanecarboxamide.

The compounds having 5HT_{1D} and 5HT_{1A} antagonist activity can be administered together or individually for the treatment of CNS disorders, that is to say either concurrently or non-concurrently.

As used herein, concurrently shall be understood to mean that the two agents are administered together or within 24 hours or less of each other, preferably within about 12 hours of each other, more preferably within about 1 hour of each other and most preferably within about 5 minutes of each other. Concurrent administration includes coadministration of separate dosage forms of the two agents or administration as a single dosage unit. Non-concurrently shall be taken to mean that the two agents are administered more than 24 hours apart.

In a further aspect of the present invention there is therefore provided a kit comprising in separate dosage forms a compound having 5HT_{1D} antagonist acitivity and a compound having 5HT_{1A} antagonist acitivity. In particular, such kits are of use in providing to patients when administration of separate doses of the two active ingredients is required. Such kits can also be provided where sequential administration of the 5HT_{1D} antagonist and 5HT_{1A} antagonist is required.

The invention also extends to pharmaceutical compositions comprising a compound having antagonist activity at both the 5HT_{1D} and 5HT_{1A} receptors, that is to say a single compound having dual activity, and a pharmaceutically acceptable carrier for the treatment or prevention of CNS disorders.

The compositions of the present invention are expected to be of use in the treatment of CNS disorders such as mood disorders, including depression, seasonal effective disorder and dysthymia; anxiety disorders, including generalised anxiety, panic disorder, agoraphobia, social phobia, obsessive compulsive disorder and post-traumatic stress disorder; memory disorders, including dementia, amnestic disorders and age-associated memory impairment; and disorders of eating behaviours, including anorexia nervosa and bulimia nervosa. Other CNS disorders include Parkinson's disease, dementia in Parkinson's disease, neuroleptic-induced Parkinsonism and tardive dyskinesias, as well as other psychiatric disorders.

The compositions of the present invention may also be of use in the treatment of endocrine disorders such as hyperprolactinaemia, in the treatment of vasospasm (particularly in the cerebral vasculature) and hypertension, as well as disorders in the gastrointestinal tract where changes in motility and secretion are involved. They may also be of use in the treatment of sexual dysfunction.

Therefore in a further aspect the present invention provides a pharmaceutical composition which comprises a compound having 5HT_{1D} antagonist acitivity, a compound having 5HT_{1A} antagonist acitivity, and a pharmaceutically acceptable carrier for use in therapy.

In another aspect the invention provides a pharmaceutical composition which comprises a compound having 5HT_{1D} antagonist acitivity, a compound having 5HT_{1A} antagonist acitivity; and a pharmaceutically acceptable carrier in the manufacture of a medicament for the treatment of the aforementioned disorders.

In particular the invention provides a pharmaceutical composition which comprises a compound having 5HT_{1D} antagonist acitivity, a compound having 5HT_{1A} antagonist acitivity; and a pharmaceutically acceptable carrier for use in the treatment or prophylaxis of depression.

Compositions of the invention can also be administered in combination with other medicaments, for example conventional antidepressants or anxiolytics.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1% to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 200 mg, and such unit doses may be administered more than once a day, for example two or three a day. Such therapy may extend for a number of weeks or months.

Preferred compounds of the invention can be prepared according to the following examples.

### Example 1

### N-[1-(2-Dimethylaminoethyl)indol-6-yl]-2'-methyl-4'-(5-methyl-1,2,4-oxadiazol-3-yl)biphenyl-4-carboxamide

2'-Methyl-4'(5-methyl-11,2,4-oxadiazol-3-yl) [1,1'-biphenyl-4-carboxylic acid) (E.P.0533268-A1) (0.19g, 0.7mmol) was suspended in CH₂Cl₂ (15ml) and treated with oxalylchloride (0.065ml, 0.074mmol) followed by a drop of DMF. The mixture was stirred at room temperature for 1hr, then evaporated under reduced pressure to give a pale yellow solid. The solid was redissolved in dichloromethane (10ml) and added to a solution of 6-amino-1-(2-dimethylaminoethyl)-1H-indole (0.14g, 0.7mmol) in CH₂Cl₂ (10ml) containing Et₃N (0.19ml, 1.4mmol) under argon. After 19hr at room temperature, the reaction mixture was treated with water (20ml),extracted with CH₂Cl₂ and the combined organic layers were dried (Na₂SO₄) and evaporated under reduced pressure to give a brown oil which was purified by flash column chromatography using CH₂Cl₂ as eluant. The title compound was isolated as a white solid. (90mg, 30%)
¹H NMR (250mHz, CDCl₃) δ : 8.40 (s, 1H) 8.20 (s, 1H) 7.99-7.90 (m, 4H), 7.53 (d, 1H), 7.39 (d, 2H), 7.30 (d, 1H), 7.12 (d, 1H), 7.08 (dd, 1H), 6.46 (d, 1H), 4.18 (t, 2H), 2.71-2.65 (m, 5H), 2.31 (s, 3H), 2.25 (s, 6H).

### Example 2

### (N-(2-(4-(2-methoxyphenyl)-1-piperazinyl)ethyl)-N-(2-pyridynyl)cyclohexanecarboxamide

The title compound was prepared according to the procedure in EPA 0 512 755.

### Evaluation of the effects of 5-HT_{1D/1A} receptor antagonists by microdialysis in concious guinea pigs

The effects of 5-HT_{1D/1A} receptor ligands on extracellular levels of 5-HT can be determined in vivo using the technique of microdialysis combined with high performance liquid chromatography and electrochemical detection (HPLC-ECD). Male Dunkin Hartley guinea pigs were anaesthetised with methoxyflurane and microdialysis probes were stereotaxically implanted into the frontal cortex (co-ordinates : 4.5 mm anterior, 2 mm lateral with reference to Bregma, lowered 3 mm from the skull surface). Microdialysis probes were secured to the skull surface using dental acrylic and the animals allowed a 24 hour recovery period. Probes were then perfused with artificial cerebrospinal fluid (aCSF) at a flow rate of 2 ul/min and samples collected every 20 min. Samples were then analysed for 5-HT and its metabolite 5-HIAA using HPLC-ECD. For data analysis 5-HT levels in the sample immediately prior to administration of drug or vehicle was taken as 100%, levels of 5-HT were then expressed as a % of this baseline level.

The following figures were obtained for 5-HT concentrations in the dialysates from frontal cortex expressed as a percentage of control, measured at the time of peak effect.

### Compound A (5-HT_{1D} antagonist)

N-[4-methoxy-3-(4-methyl-1-piperazinyl)phenyl]-2'-methyl-4'-(5-methyl-1,2,4-oxadiazol-3-yl)[1,1'-biphenyl]-4-carboxamide:
0.3 mg/kg ip: -31 +/-12 (n=6)

### Compound B (5-HT_{1A} antagonist)

(N-(2-(4-(2-methoxyphenyl)-1-piperazinyl)ethyl)-N-(2-pyridynyl)cyclohexanecarboxamide:
1mg/kg ip: +20 +/-9 (n=5)

### Compound A + Compound B

+405 +/-228 (n=5)

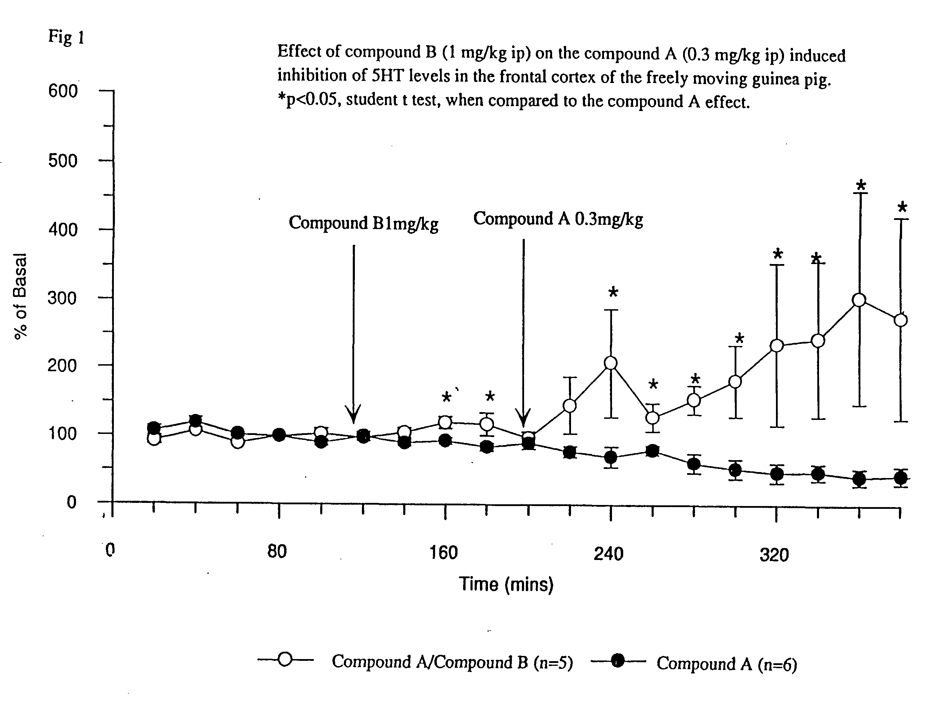

The results are shown graphically in Figure 1.

## Claims

1. A pharmaceutical composition for use in therapy which comprises:
• a compound having 5HT_{1D} antagonist activity;
• a compound having 5HT_{1A} antagonist activity; and
• a pharmaceutically acceptable carrier.

2. A pharmaceutical composition according to claim 1 in which the 5HT_{1D} antagonist is:
N-[4-methoxy-3-(4-methyl-1-piperazinyl)phenyl]-2'-methyl-4'-(5-methyl-1,2,4-oxadiazol-3-yl)[1,1'-biphenyl]-4-carboxamide,
or a pharmaceutically acceptable salt thereof.

3. A pharmaceutical composition according to claim 1 or 2 in which the 5HT_{1A} antagonist is:
(+)-2,3,4,5,6,7-hexahydro-1-(4-(1-(1,2,3,6-tetrahydro-4-(2-methoxyphenyl)pyridyl))-2-phenyl-butyryl)-1H-azepine,
1-(1H-indol-4-yloxy)-3-[(1-methylethyl)amino]-2-propanol,
(*S*)-5-fluoro-8-hydroxy-2-(dipropylamino)-tetralin,
N-tert-butyl 3-4-(2-methoxyphenyl) piperazin-1-yl-2-phenylpropanamide,
(N-(2-(4-(2-methoxyphenyl)-1-piperazinyl)ethyl)-N-(2-pyridynyl)cyclohexanecarboxamide,
or a pharmaceutically acceptable salt thereof.

4. A pharmaceutical composition comprising a single compound having antagonist activity at both the 5HT_{1D} and 5HT_{1A} receptors and a pharmaceutically acceptable carrier.

5. A pharmaceutical composition according to any one of claims 1 to 4 for use in the treatment or prevention of CNS disorders.

6. A pharmaceutical composition according to any one of claims 1 to 4 for use in the treatment or prevention of depression.

7. The use of a pharmaceutical composition according to any one of claims 1 to 4 in the manufacture of a medicament for use in the treatment of CNS disorders.

8. A kit comprising a dosage unit containing a 5HT_{1A} antagonist or a pharmaceutically acceptable salt thereof and a dosage unit containing a 5HT_{1D} antagonist or a pharmaceutically acceptable salt thereof.

## Patentansprüche

1. Arzneimittel zur Verwendung bei der Therapie, welches
eine Verbindung mit Wirksamkeit als 5HT_{1D}-Antagonist;
eine Verbindung mit Wirksamkeit als 5HT_{1A}-Antagonist; und
einen pharmazeutisch verträglichen Träger umfaßt.

2. Arzneimittel nach Anspruch 1, bei welchem der 5HT_{1D}-Antagonist
N-[4-Methoxy-3-(4-methyl-1-piperazinyl)phenyl]-2'-methyl-4'-(5-methyl-1,2,4-oxadiazol-3-yl)[1,1'-biphenyl]-4-carboxamid
oder ein pharmazeutisch verträgliches Salz davon ist.

3. Arzneimittel nach Anspruch 1 oder 2, bei welchem der 5HT_{1A}-Antagonist
(+)-2,3,4,5,6,7-Hexahydro-1-(4-(1-(1,2,3,6-tetrahydro-4-(2-methoxyphenyl)pyridyl))-2-phenyl-butyryl)- 1H-azepin,
1-(1H-Indol-4-yloxy)-3-[(1-methylethyl)amino]-2-propanol,
(S)-5-Fluor-8-hydroxy-2-(dipropylamino)-tetralin,
N-tert-Butyl-3-4-(2-methoxyphenyl)piperazin-1-yl-2-phenylpropanamid,
(N-(2-(4-(2-Methoxyphenyl)-1-piperazinyl)ethyl)-N-(2-pyridinyl)cyclohexancarboxamid
oder ein pharmazeutisch verträgliches Salz davon ist.

4. Arzneimittel umfassend eine einzige Verbindung mit antagonistischer Wirksamkeit sowohl an dem 5HT_{1D}- als auch an dem 5HT_{1A}-Rezeptor und einen pharmazeutisch verträglichen Träger.

5. Arzneimittel nach einem der Ansprüche 1 bis 4 zur Verwendung bei der Behandlung oder Prävention von Störungen des Zentralnervensystems.

6. Arzneimittel nach einem der Ansprüche 1 bis 4 zur Verwendung bei der Behandlung oder Prävention von Depressionen.

7. Verwendung eines Arzneimittels nach einem der Ansprüche 1 bis 4 bei der Herstellung eines Medikaments zur Verwendung bei der Behandlung von Störungen des Zentralnervensystems.

8. Packung umfassend eine Dosierungseinheit, die einen 5HT_{1A}-Antagonisten oder ein pharmazeutisch verträgliches Salz davon enthält, und eine Dosierungseinheit, die einen 5HT_{1D}-Antagonisten oder ein pharmazeutisch verträgliches Salz davon enthält.

## Revendications

1. Composition pharmaceutique destinée à être utilisée en thérapeutique, qui comprend :
• un composé ayant une activité antagoniste de 5HT_{1D} ;
• un composé ayant une activité antagoniste de 5HT_{1A} ; et
• un support pharmaceutiquement acceptable.

2. Composition pharmaceutique suivant la revendication 1, dans laquelle l'antagoniste de 5HT_{1D} est :
le N-[4-méthoxy-3-(4-méthyl-1-pipérazinyl)phényl]-2'-méthyl-4'-(5-méthyl-1,2,4-oxadiazole-3-yl) [1,1'-biphényl]-4-carboxamide,
ou un de ses sels pharmaceutiquement acceptables.

3. Composition pharmaceutique suivant la revendication 1 ou 2, dans laquelle l'antagoniste de 5HT_{1A} est :
la (+)-2,3,4,5,6,7-hexahydro-1-(4-(1-(1,2,3,6-tétrahydro-4-(2-méthoxyphényl)pyridyl))-2-phényl-butyryl)-1H-azépine,
le 1-(1H-indole-4-yloxy)-3-[(1-méthyléthyl)amino]-2-propanol,
la (S)-5-fluoro-8-hydroxy-2-(dipropylamino)-tétraline,
le N-tertio-butyl-3,4-(2-méthoxyphényl)pipérazine-1-yl-2-phénylpropanamide,
le (N-(2-(4-(2-méthoxyphényl)-1-pipérazinyl)éthyl)-N-(2-pyridynyl)cyclohexane-carboxamide,
ou un de leurs sels pharmaceutiquement acceptables.

4. Composition pharmaceutique comprenant un composé unique ayant une activité antagoniste à la fois au niveau des récepteurs 5HT_{1D} et 5HT_{1A} et un support pharmaceutiquement acceptable.

5. Composition pharmaceutique suivant l'une quelconque des revendications 1 à 4, destinée à être utilisée dans le traitement ou la prévention de troubles du système nerveux central.

6. Composition pharmaceutique suivant l'une quelconque des revendications 1 à 4, destinée à être utilisée dans le traitement ou la prévention de la dépression.

7. Utilisation d'une composition pharmaceutique suivant l'une quelconque des revendications 1 à 4 dans la production d'un médicament destiné à être utilisé dans le traitement de troubles du système nerveux central.

8. Kit comprenant une unité posologique contenant un antagoniste de 5HT_{1A} ou un de ses sels pharmaceutiquement acceptables et une unité posologique contenant un antagoniste de 5HT_{1D} ou un de ses sels pharmaceutiquement acceptables.
